# EUROPEAN PATENT APPLICATION

(11) **EP 4 427 744 A1**
(43) Date of publication of application: **11.09.2024**
(21) Application number: 22889387.1
(22) Date of filing: 03.11.2022
(51) Int. Cl.: A61K 9/70, A61K 31/496, A61P 25/18

(54) **LURASIDONE HYDROCHLORIDE ORAL SOLUBLE FILM COMPOSITION, PREPARATION METHOD THEREFOR AND USE THEREOF**

(30) Priority: 04.11.2021 CN 202111301406
(71) Applicant: Shanghai Aurora Biotechnology Co., Ltd., Shanghai 201210 (CN)
(72) Inventor: GUO, Zhen, Shanghai 201210 (CN); FU, Jun, Shanghai 201210 (CN); NI, Hui, Shanghai 201210 (CN); LU, Pengcheng, Shanghai 201210 (CN); LIU, Yuli, Shanghai 201210 (CN); WANG, Tingting, Shanghai 201210 (CN); YING, Shuhuan, Shanghai 201210 (CN)
(74) Representative: Gille Hrabal Partnerschaftsgesellschaft mbB Patentanwälte
(86) International application number: PCT/CN2022/129623
(87) International publication number: WO 2023/078366

(57) **Abstract**

Provided are a lurasidone hydrochloride oral soluble film composition, a preparation method therefor and a use thereof. The lurasidone hydrochloride oral soluble film composition contains an active drug, a film-forming material, a plasticizer and a flavoring agent. The active drug is (3aR,4S,7R,7aS)-2-((1R,2R)-2-[4-(1.2-benzisothiazole 3-yl)piperazine-1-methyl]cyclohexylmethyl)hexahydro-4.7-methylene-2H-isoindole-1,3-dione hydrochloride, as shown in Formula I. The obtained lurasidone hydrochloride oral soluble film composition has a good dissolution rate, has no gravelly feeling in the oral cavity after dissolution, has a uniform appearance and good flexibility, and does not precipitate during the preparation of the film liquid; content uniformity thereof meets requirements.

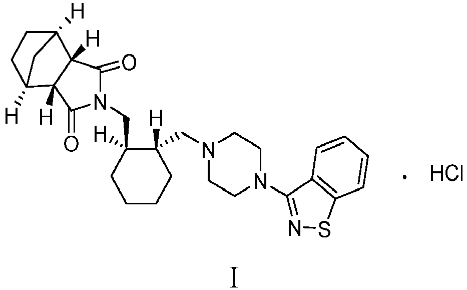

## Description

The present application claims priority to the prior application with the patent application No. 202111301406.X titled "LURASIDONE HYDROCHLORIDE ORAL SOLUBLE FILM COMPOSITION, PREPARATION METHOD THEREFOR AND USE THEREOF" filed to the China National Intellectual Property Administration on November 4, 2021, the contents of which is incorporated herein by reference in its entirety.

### TECHNICAL FIELD

The present disclosure relates to the field of pharmaceutical formulations and in particular to a lurasidone hydrochloride oral soluble film composition featuring simple process and good stability, a preparation method therefor, and use thereof.

### BACKGROUND

Schizophrenia is a serious mental disorder. According to the statistics of the World Health Organization, more than 21 million people are suffering from schizophrenia in the world. This disease is also the eighth leading cause of disability among the 15-44 year old. By the end of 2016, the number of schizophrenia patients registered in China was about 4.05 million. The existing drugs for schizophrenia treatment can be roughly divided into two categories. One is typical antipsychotic drugs, also known as the first-generation antipsychotic drugs, and the other is atypical antipsychotic drugs which generally have dopamine D2/5-HT2A receptor antagonism and are also known as the second-generation antipsychotic drugs. Since 2010, the market scale of mental drugs in the domestic sample hospitals has increased from 1.25 billion yuan to 2.47 billion yuan in 2015, with the 5-year compound annual growth rate reaching 14.6%. In view of the incomplete understanding of the pathogenesis of schizophrenia and the limitations in the efficacy of existing therapeutic drugs as well as the adverse effects of the therapeutic drugs, therapeutic drugs with better efficacy and less severe adverse effects are needed in clinic. The medical resources of the domestic psychiatric department are short, and the potential of the mental drug market is huge. However, in the acute phase, schizophrenic patients often refuse to take the medicine, store the medicine in the oral cavity, and spit the medicine, and the problem of medication compliance among patients is common. Thus, tablets and capsules are often ineffective for patients, and dosage forms that are easy to use are considered for schizophrenic patients. In view of the above, there is a need to provide a new formulation featuring convenient use, good compliance, and stable quality to solve the above problems.

Lurasidone hydrochloride is a novel atypical antipsychotic drug jointly developed by Takeda Pharmaceuticals and Sumitomo Pharma for treating schizophrenia, and it is used for treating schizophrenia and bipolar depression. At present, the clinical dosage form is a common tablet. However, it is difficult for the common tablet to be swallowed, and this dosage form is not suitable for children and elderly patients and cannot be taken by the patients conveniently without water.

Therefore, a dosage form of lurasidone hydrochloride needs to be developed at present to solve the problem of poor compliance of patients in taking medicines, and this dosage form should be particularly suitable for patients with dysphagia and thus can improve compliance of the patients.

### SUMMARY

The present disclosure provides a lurasidone hydrochloride oral soluble film composition, which comprises an active drug, a film-forming material, a plasticizer, and a flavoring agent, wherein the active drug is a compound represented by formula I, namely (3*aR*,4*S*,7*R*,7*aS*)-2-((1*R*,2*R*)-2-[4-(1.2-benzisothiazol-3-yl)plperazin-1-methyl] cyclohexylmethyl)hexahydro-4.7-methano-2*H*-isoindole-1,3-dione hydrochloride (lurasidone hydrochloride);

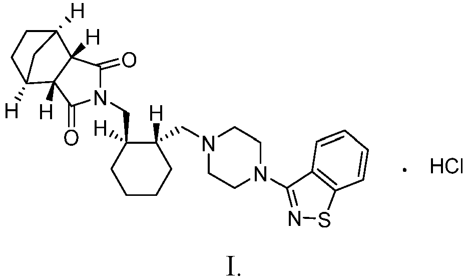

According to an embodiment of the present disclosure, a mass percentage content of the active drug is preferably 1%-60%, such as 10%-55%, for example 10%-50%, e.g., 52.1%, 47.2%, 41.7%, 41.3%, 38.5%, 35.7%, 33.3%, 30.0%, or 25.0%, wherein the mass percentage content refers to the percentage of the mass of the active drug relative to the total mass of the lurasidone hydrochloride oral soluble film composition.

According to an embodiment of the present disclosure, the film-forming material is a carrier of the drug and is preferably one or more of xanthan gum, guar gum, pectin, gelatin, shellac, acacia, starch, dextrin, agar, sodium alginate, zein, hydroxypropyl methylcellulose, hydroxymethylcellulose, hydroxyethylcellulose, hydroxypropylcellulose, polyvinyl alcohol, polyvinylpyrrolidone, polyethylene glycol, polyoxyethylene, pullulan, acrylic acid copolymer, polylactic acid, and silicone rubber. In some embodiments, the film-forming material is polyvinyl alcohol, hydroxypropylcellulose, a mixture of polyvinyl alcohol and polyethylene glycol 6000, or a mixture of hydroxyethylcellulose and pullulan.

According to an embodiment of the present disclosure, a mass percentage content of the film-forming material is 20%-60%, such as 30%-50%, e.g., 57.0%, 50.0%, 47.0%, 45.8%, 41.7%, 41.3%, 38.5%, 37.7%, 35.7%, 33.0%, or 31.2%, wherein the mass percentage content refers to the percentage of the mass of the film-forming material relative to the total mass of the lurasidone hydrochloride oral soluble film composition.

According to an embodiment of the present disclosure, the plasticizer is used for reducing the glass transition temperature of the film, increasing the plasticity and toughness, and improving the stretching rate, and is preferably one or more of polyethylene glycol, glycerol, propylene glycol, silicone oil, polypropylene glycol, and hexanediol.

According to an embodiment of the present disclosure, a mass percentage content of the plasticizer is 0-20%, e.g., 16.0%, 9.4%, 8.3%, 5.2%, 5.0%, 4.7%, 4.5%, 4.3%, or 0, wherein the mass percentage content refers to the percentage of the mass of the plasticizer relative to the total mass of the lurasidone hydrochloride oral soluble film composition.

According to an embodiment of the present disclosure, the flavoring agent plays a flavoring role in the film formulation and is preferably one or more of aspartame, sucralose, fructose, sucrose, stevioside, glycyrrhizin, essence, spice, menthol, sodium chloride, citric acid, saccharin, and saccharin sodium.

According to an embodiment of the present disclosure, a mass percentage content of the flavoring agent is 0-25.0%, preferably 3.0%-25.0%, e.g., 17.7%, 17.1%, 16.4%, 12.5%, 10.4%, 10.0%, 9.5%, 9.4%, 8.3%, 5.0%, or 3.0%, wherein the mass percentage content refers to the percentage of the mass of the flavoring agent relative to the total mass of the lurasidone hydrochloride oral soluble film composition. According to an embodiment of the present disclosure, the lurasidone hydrochloride oral soluble film composition may further comprise a disintegrant or a combination of a disintegrant and one or more of a saliva stimulant, a filler, a colorant, an anti-adherent, and a bacteriostatic agent.

According to an embodiment of the present disclosure, the disintegrant refers to an auxiliary material for promoting rapid disintegration of a drug into small particles in the gastrointestinal tract and is preferably one or more of low-substituted hydroxypropylcellulose, crospovidone, croscarmellose sodium, sodium carboxymethyl starch, starch, microcrystalline cellulose, and pregelatinized starch. According to an embodiment of the present disclosure, a mass percentage content of the disintegrant is preferably 0-10.0%, e.g., 3.6%, 6.9%, 7.0%, or 7.1%, wherein the mass percentage content refers to the percentage of the mass of the disintegrant relative to the total mass of the lurasidone hydrochloride oral soluble film composition.

According to an embodiment of the present disclosure, the saliva stimulant refers to a substance for stimulating saliva production and is preferably one or more of tartaric acid, malic acid, and mannitol.

According to an embodiment of the present disclosure, a mass percentage content of the saliva stimulant is 0-10.0%, e.g., 0.6%, 0.9%, 6.9%, 7.0%, 7.1%, or 7.7%, wherein the mass percentage content refers to the percentage of the mass of the saliva stimulant relative to the total mass of the lurasidone hydrochloride oral soluble film composition.

According to an embodiment of the present disclosure, the filler refers to a solid substance capable of improving the performance of a material, increasing the volume and weight, and reducing the cost of the material when added to the material and is preferably one or more of mannitol, sucrose, glucose, maltose, lactose, sorbitol, xylitol, maltitol, galactitol, erythritol, dextrin, and trehalose.

According to an embodiment of the present disclosure, a mass percentage content of the filler is 0-10.0%, e.g., 6.0% or 3.0%, wherein the mass percentage content refers to the percentage of the mass of the filler relative to the total mass of the lurasidone hydrochloride oral soluble film composition.

According to an embodiment of the present disclosure, the colorant refers to a substance that is capable of improving the appearance and color of a formulation and can be used for identifying the concentration of the formulation, distinguishing the application method, and reducing the patient's aversion to taking medicines and is preferably one or more of titanium dioxide, pigment, and lake.

According to an embodiment of the present disclosure, a mass percentage content of the colorant is 0-5.0%, e.g., 1.1%, 1.0%, or 0.8%, wherein the mass percentage content refers to the percentage of the mass of the colorant relative to the total mass of the lurasidone hydrochloride oral soluble film composition.

According to an embodiment of the present disclosure, the anti-adherent refers to a substance capable of improving the performance of a formulation and avoiding the adhesion between formulations and is preferably one or more of talc, magnesium stearate, and calcium stearate.

According to an embodiment of the present disclosure, a mass percentage content of the anti-adherent is 0-5.0%, e.g., 0.7% or 0.8%, wherein the mass percentage content refers to the percentage of the mass of the anti-adherent relative to the total mass of the lurasidone hydrochloride oral soluble film composition.

According to an embodiment of the present disclosure, the bacteriostatic agent refers to a substance capable of inhibiting the production of bacteria and is preferably one or more of methylparaben, ethylparaben, and sodium thiosulfate.

According to an embodiment of the present disclosure, a mass percentage content of the bacteriostatic agent is 0-1.0%, e.g., 0.6%, wherein the mass percentage content refers to the percentage of the mass of the bacteriostatic agent relative to the total mass of the lurasidone hydrochloride oral soluble film composition.

According to an embodiment of the present disclosure, the lurasidone hydrochloride oral soluble film composition may be any one of the following formulas:
formula 1: 47.2% lurasidone hydrochloride, 33.0% hydroxypropylcellulose, 9.4% glycerol, 9.4% sucralose, and 1.0% titanium dioxide;
formula 2: 30.0% lurasidone hydrochloride, 45.0% polyvinyl alcohol, 12.0% polyethylene glycol 6000, 5.0% sucralose, 3.5% menthol, 0.9% mannitol, 3.0% lactose, and 0.6% methylparaben;
formula 3: 33.3% lurasidone hydrochloride, 45.8% polyvinyl alcohol, 8.3% glycerol, 3.3% sodium chloride, 4.2% citric acid, 4.3% sucralose, 0.4% essence, and 0.4% menthol;
formula 4: 25.0% lurasidone hydrochloride, 50.0% polyvinyl alcohol, 16.0% glycerol, 2.5% stevioside, 0.5% essence, and 6.0% lactose;
formula 5: 41.3% lurasidone hydrochloride, 24.8% hydroxyethylcellulose, 16.5% pullulan, 8.3% glycerol, 8.3% sucralose, and 0.8% titanium dioxide;
formula 6: 47.2% lurasidone hydrochloride, 28.3% hydroxyethylcellulose, 9.4% pullulan, 4.7% glycerol, 9.4% sucralose, and 1.0% titanium dioxide;
formula 7: 52.1% lurasidone hydrochloride, 20.8% hydroxyethylcellulose, 10.4% pullulan, 5.2% glycerol, 10.4% sucralose, and 1.1% titanium dioxide;
formula 8: 41.7% lurasidone hydrochloride, 25.0% hydroxyethylcellulose, 16.7% pullulan, 5.0% glycerol, 8.3% sucralose, 1.7% menthol, 0.8% talc, and 0.8% titanium dioxide;
formula 9: 38.5% lurasidone hydrochloride, 23.1% hydroxyethylcellulose, 15.4% pullulan, 4.5% glycerol, 7.7% sucralose, 1.5% menthol, 0.8% essence, 7.7% citric acid, and 0.8% talc;
formula 10: 35.7% lurasidone hydrochloride, 21.4% hydroxyethylcellulose, 14.3% pullulan, 4.3% glycerol, 7.1% sodium carboxymethyl starch, 7.1% sucralose, 1.4% menthol, 0.8% essence, 7.1% citric acid, and 0.8% talc.

The present disclosure further provides a method for preparing the lurasidone hydrochloride oral soluble film composition, which comprises the following steps:
1) dissolving the film-forming material in water by heating at 60-70 °C to form a solution;
2) adding other components except for the active drug and the film-forming material into the solution obtained in step 1) and stirring homogeneously to obtain a blank gel solution;
3) placing the active drug in the blank gel solution obtained in step 2), stirring until the active drug is evenly dispersed, and defoaming by stirring under vacuum to obtain a drug-containing gel solution; and
4) evenly coating the drug-containing gel on a release film by using a coating machine, heating, drying, and cutting to obtain the lurasidone hydrochloride oral soluble film composition.

According to an embodiment of the present disclosure, the thickness of the lurasidone hydrochloride oral soluble film composition is 10-300 µm, e.g., 10-300 µm. According to an embodiment of the present disclosure, the lurasidone hydrochloride oral soluble film composition is capable of being completely disintegrated in 1000 mL of simulated saliva at 37±1 °C within 2 min.

The present disclosure further provides use of the lurasidone hydrochloride oral soluble film composition for the manufacturing of a medicament for the treatment and/or prevention of depression.

The present disclosure further provides a method for treating and/or preventing depression, which comprises administering to a patient in need thereof a therapeutically effective amount of the lurasidone hydrochloride oral soluble film composition.

According to an embodiment of the present disclosure, the lurasidone hydrochloride oral soluble film composition is a pharmaceutical formulation.

Beneficial effects of the present disclosure are as follows. The lurasidone hydrochloride oral soluble film composition of the present disclosure has the advantages of thin thickness, good taste, stable properties, good dissolution rate, instant dissolution in the oral cavity without drinking water, no gritty taste after dissolution in the oral cavity, and high oral absorption speed. Further, it features uniform appearance, good flexibility, simple process, no precipitation during the preparation of the film liquid, up-to-standard content uniformity, and high drug-loading rate. The lurasidone hydrochloride oral soluble film composition of the present disclosure overcomes the defects of inconvenience in taking, poor patient compliance, and the like of the existing formulations and is particularly suitable for patients with dysphagia.

### Definitions and Description

Unless otherwise stated, the definitions of terms described in the specification and claims of the present application, including definitions thereof as examples, exemplary definitions, preferred definitions, specific definitions in the examples, and the like, may be arbitrarily combined and incorporated with each other. Such combinations and incorporation shall fall within the scope of the present specification.

The term "therapeutically effective amount" refers to an amount of the active pharmaceutical ingredient of the present disclosure sufficient to effect the intended use (including but not limited to the treatment of a disease as defined below). The therapeutically effective amount may vary depending on the following factors: the intended use (*in vitro* or *in vivo*), or the subject and diseases or conditions being treated, such as the body weight and age of the subject, severity of the diseases or conditions, route of administration, and the like, which may be readily determined by one of ordinary skill in the art. The specific dose will vary depending on the following factors: the selected particular active ingredient, the dosage regimen to be followed, whether to administer in combination with other compounds, the schedule of administration, the tissue to be administered, and the physical delivery system carried.

### BRIEF DESCRIPTION OF THE DRAWINGS

FIG. 1 is the dissolution profile of the lurasidone hydrochloride oral soluble film formulation of Example 3 in a hydrochloric acid solution at pH 1.2; and
FIG. 2 is the dissolution profile of the lurasidone hydrochloride oral soluble film formulation of Example 3 in an acetate buffer solution at pH 3.8.

### DETAILED DESCRIPTION

The technical solutions of the present disclosure will be further described in detail with reference to the following specific examples. It should be understood that the following examples are merely exemplary illustration and explanation of the present disclosure and should not be construed as limiting the protection scope of the present disclosure. All techniques implemented based on the aforementioned content of the present disclosure are encompassed within the protection scope of the present disclosure.

Unless otherwise stated, the starting materials and reagents used in the following examples are all commercially available products or can be prepared by using known methods. Experimental procedures without specified conditions in the following examples are conducted in accordance with conventional procedures and conditions, or in accordance with the manufacturer's manual.

### Example 1

The formulas are shown in Table 1:

**Table 1**

| Examples | | Example 1 | Example 2 | Example 3 | Example 4 | Example 5 | Example 6 | Example 7 | Example 8 | Example 9 | Example 10 |
|---|---|---|---|---|---|---|---|---|---|---|---|
| Drug substance | Lurasidone hydrochloride | 5g (47.2%) | 5.1g (30.0%) | 5g (33.3%) | 5g (25.0%) | 5g (41.3%) | 5g (47.2%) | 5g (52.1%) | 5g (41.7%) | 5g (38.5%) | 5g (35.7%) |
| Film-formin g material | Polyvinyl alcohol | / | 7.7g (45.0%) | 6.88g (45.8%) | 10g (50.0%) | / | / | / | / | / | / |
| | Hydroxyethylcellulose | / | / | / | / | 3g (24.8%) | 3g (28.3%) | 2g (20.8%) | 3g (25%) | 3g (23.1%) | 3g (21.4%) |
| | Hydroxypropylcellulose | 3.5g (33.0%) | / | / | / | / | / | / | / | / | / |
| | Pullulan | / | / | / | / | 2g (16.5%) | 1g (9.4%) | 1g (10.4%) | 2g (16.7%) | 2g (15.4%) | 2g (14.3%) |
| | Polyethylene glycol 6000 | / | 2g (12.0%) | / | / | / | / | / | / | / | / |
| Plasticizer | Glycerol | 1g (9.4%) | / | 1.25g (8.3%) | 3.2g (16%) | 1g (8.3%) | 0.5g (4.7%) | 0.5g (5.2%) | 0.6g (5.0%) | 0.6g (4.5%) | 0.6g (4.3%) |
| Disintegrant | Sodium carboxymethyl starch | / | / | / | / | / | / | / | / | / | 1g (7.1%) |
| Flavoring agent | Sucralose | 1g (9.4%) | 0.9g (5.0%) | 0.63g (4.3%) | / | 1g (8.3%) | 1g (9.4%) | 1g (10.4%) | 1g (8.3%) | 1g (7.7%) | 1g (7.1%) |
| | Stevioside | / | / | / | 0.5g (2.5%) | / | / | / | / | / | / |
| | Sodium chloride | / | / | 0.5g (3.3%) | / | / | / | / | / | / | / |
| | Menthol | / | 0.6g (3.5%) | 0.06g (0.4%) | / | / | / | / | 0.2g (1.7%) | 0.2g (1.5%) | 0.2g (1.4%) |
| | Essence | / | / | 0.06g (0.4%) | 0.1g (0.5%) | / | / | / | / | 0.1g (0.8%) | 0.1g (0.8%) |
| | Citric acid | / | / | 0.63g (4.2%) | / | / | / | / | / | 1g (7.7%) | 1g (7.1%) |
| Anti-adherent | Talc | / | / | / | / | / | / | / | 0.1g (0.8%) | 0.1g (0.8%) | 0.1g (0.8%) |
| Disintegrant | Sodium carboxymethyl starch | / | / | / | / | / | / | / | / | / | / |
| Saliva stimulant | Mannitol | / | 0.2g (0.9%) | / | / | / | / | / | / | / | / |
| Filler | Lactose | / | 0.5g (3.0%) | / | 1.2g (6.0%) | / | / | / | / | / | / |
| Colorant | Titanium dioxide | 0.1g (1.0%) | / | / | / | 0.1g (0.8%) | 0.1g (1.0%) | 0.1g (1.1%) | 0.1g (0.8%) | / | / |
| Bacteriostatic agent | Methylparaben | / | 0.1g (0.6%) | / | / | / | / | / | / | / | / |
| Purified water* | | 170 | 230 | 200 | 280 | 160 | 150 | 150 | 170 | 170 | 160 |
| / | Total weight** | 10.6g | 17.1g | 15.0g | 20.1g | 12.1g | 10.6g | 9.6g | 12.0g | 13.0g | 14.0g |

| | | | | | | | | | | | |
|---|---|---|---|---|---|---|---|---|---|---|---|
| *removal during the process; **the weight of the drug substance and the auxiliary materials after removal of water; the percentage of each component = the mass of each component/(total weight**) × 100%. | | | | | | | | | | | |

### Preparation method comprises:

1) dissolving the film-forming material in water by heating at 60-70 °C to form a solution;
2) adding other components except for the active drug and the film-forming material into the solution obtained in step 1) and stirring homogeneously to obtain a blank gel solution;
3) placing the active drug in the blank gel solution obtained in step 2), stirring until the active drug is evenly dispersed, and defoaming by stirring under vacuum to obtain a drug-containing gel solution; and
4) evenly coating the defoamed drug-containing gel solution obtained in step 3) on a release film by using a coating machine, heating, drying, and cutting to obtain the lurasidone hydrochloride oral soluble film composition.

### 1. Disintegration time test

Lurasidone oral soluble film formulations were prepared based on the formulas of Examples 1 to 10 and the preparation method provided by the present disclosure, and the disintegration time thereof was determined. The specific determination method is as follows:
Six drug films were randomly taken from drug films obtained for each example; at each test, 1 drug film was slightly placed in 1000 mL of artificial saliva at 37±1 °C and left to stand, and the time for the product to completely disintegrate was observed. The results are shown in Table 2.

**Table 2**

| Examples | Disintegration time (s) |
|---|---|
| 1 | 47±9 |
| 2 | 51±12 |
| 3 | 63±6 |
| 4 | 53±4 |
| 5 | 67±15 |
| 6 | 49±9 |
| 7 | 44±6 |
| 8 | 52±5 |
| 9 | 54±8 |
| 10 | 27±9 |

### 2. Determination of dissolution results

The dissolution profile of the lurasidone hydrochloride oral soluble film formulation prepared according to the formula of Example 3 was determined. The specific determination method is as follows:
test medium: 900 mL of a hydrochloric acid solution at pH 1.2 (37±0.5 °C) and 900 mL of an acetate buffer solution at pH 3.8 (37±0.5 °C).
dissolution method: 0931 Dissolution and Drug Release Test, Method 2 (Paddle), *Pharmacopoeia of the People's Republic of China (2020),* with a rotation speed of 50 rpm.
Sampling time: 5 min, 10 min, 15 min, 20 min, 30 min.

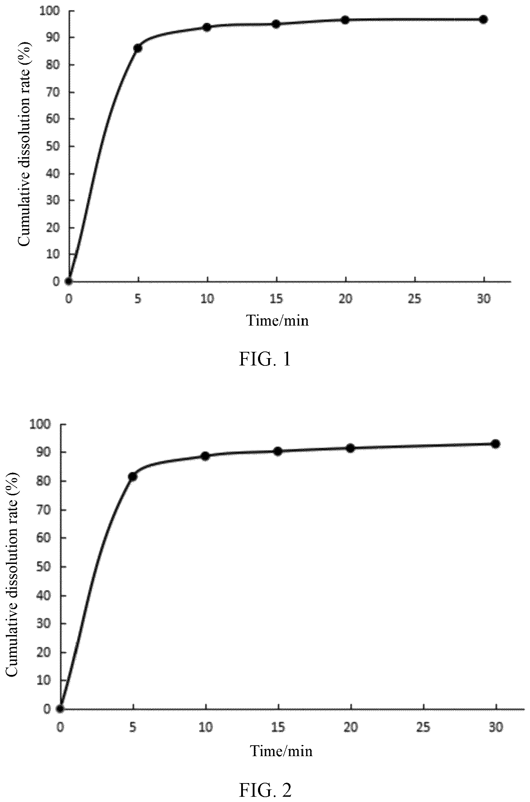

Six pieces of the lurasidone hydrochloride oral soluble film formulation were used, and the dissolution profile was determined according to the above method. The results are shown in Table 3 and FIGs. 1-2.

**Table 3**

| Conditions of medium | Sampling time | 5min | 10min | 15min | 20min | 30min |
|---|---|---|---|---|---|---|
| Hydrochloric acid solution at pH 1.2 | Cumulative dissolution rate | 86.0% | 93.7% | 94.9% | 96.4% | 96.6% |
| | RSD | 2.70% | 2.46% | 2.51% | 2.45% | 2.42% |
| Conditions of medium | Sampling time | 5min | 10min | 15min | 20min | 30min |
| Acetate buffer solution at pH 3.8 | Cumulative dissolution rate | 81.4% | 88.6% | 90.3% | 91.4% | 92.9% |
| | RSD | 4.96% | 2.87% | 2.45% | 2.45% | 2.60% |

### 3. Results for related substances

The method for detecting related substances of the lurasidone hydrochloride oral soluble film prepared according to the formula of Example 3 is as follows:

### 3.1 Description of method for analyzing related substances

### 1) Chromatographic conditions

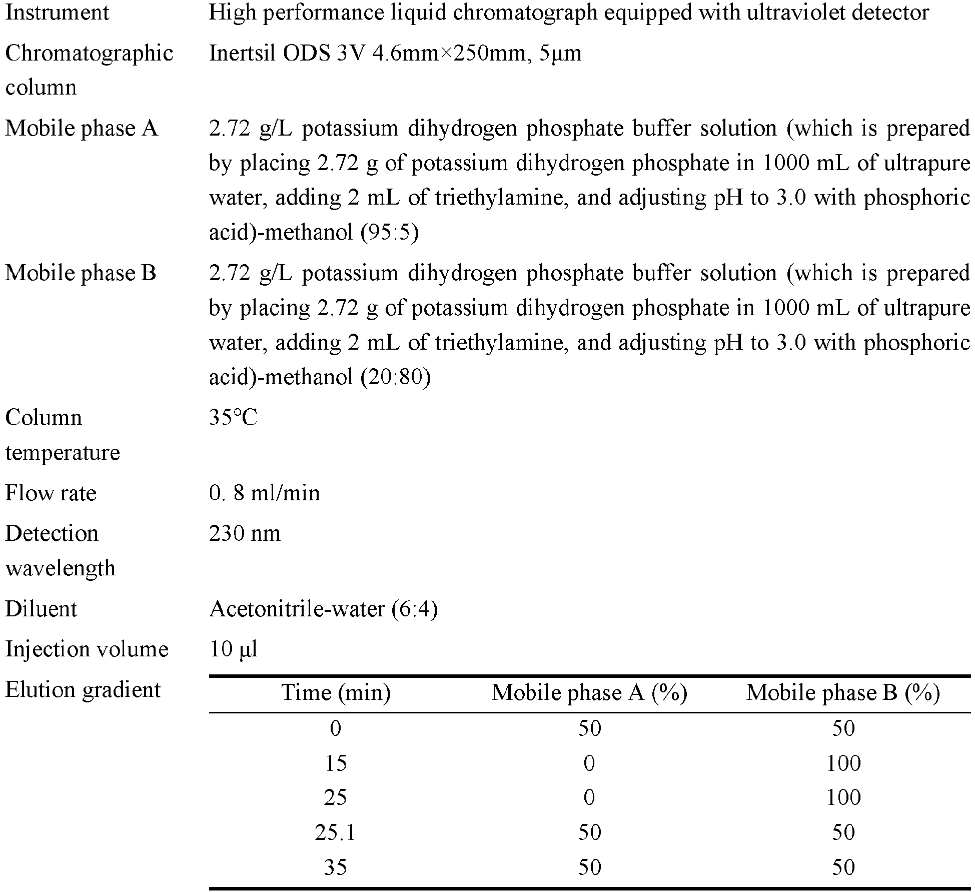

### 2) Calculation

For impurities IM-2 and IM-4, the calculation was carried out according to the external standard method for main components with a correction factor added. For unknown impurities, the calculation was carried out according to the external standard method for main components without a correction factor. The total impurities were the sum of all the known impurities and unknown impurities.

### 3) Limit of impurities

| Name | IM-4 | IM-2 | Unknown single impurities | Total impurities |
|---|---|---|---|---|
| Limit (%) | 0.25 | 0.25 | 0.2 | 0.4 |

The detection results of related substances in the sample of Example 3 are shown in Table 4.

**Table 4**

| Sample information | Maximum unknown single impurity | Total impurities |
|---|---|---|
| Lurasidone hydrochloride oral soluble film | 0.05% | 0.06% |

According to the above experimental data, the lurasidone hydrochloride oral soluble film composition provided by the present disclosure has the advantages of thin thickness, good taste, stable properties, low impurity content, instant dissolution in the oral cavity without drinking water, and high oral absorption speed.

The embodiments of the present disclosure have been described above. However, the present disclosure is not limited to the embodiments described above. Any modification, equivalent replacement, improvement, and the like made without departing from the spirit and principle of the present disclosure shall fall within the protection scope of the present disclosure.

## Claims

1. A lurasidone hydrochloride oral soluble film composition, comprising an active drug, a film-forming material, a plasticizer, and a flavoring agent, wherein the active drug is a compound represented by formula I, namely (3*aR*,4*S*,7*R*,7*aS*)-2-((1*R*,2*R*)-2-[4-(1.2-benzisothiazol-3-yl)plperazin-1-methyl] cyclohexylmethyl)hexahydro-4.7-methano-2*H*-isoindole-1,3-dione hydrochloride;

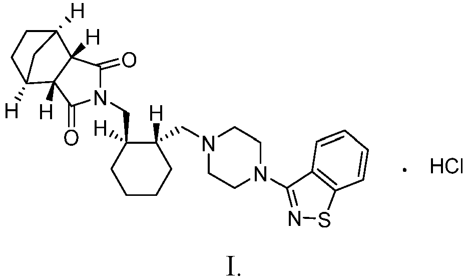

2. The lurasidone hydrochloride oral soluble film composition according to claim 1, wherein a mass percentage content of the active drug is 1%-60%, wherein the mass percentage content refers to the percentage of the mass of the active drug relative to the total mass of the lurasidone hydrochloride oral soluble film composition.

3. The lurasidone hydrochloride oral soluble film composition according to claim 1 or 2, wherein
the film-forming material is one or more of xanthan gum, guar gum, pectin, gelatin, shellac, acacia, starch, dextrin, agar, sodium alginate, zein, hydroxypropyl methylcellulose, hydroxymethylcellulose, hydroxyethylcellulose, hydroxypropylcellulose, polyvinyl alcohol, polyvinylpyrrolidone, polyethylene glycol, polyoxyethylene, pullulan, acrylic acid copolymer, polylactic acid, and silicone rubber;
and/or,
the plasticizer is one or more of polyethylene glycol, glycerol, propylene glycol, silicone oil, polypropylene glycol, and hexanediol;
and/or,
the flavoring agent is one or more of aspartame, sucralose, fructose, sucrose, stevioside, glycyrrhizin, essence, spice, menthol, sodium chloride, citric acid, saccharin, and saccharin sodium.

4. The lurasidone hydrochloride oral soluble film composition according to any one of claims 1 to 3, wherein
a mass percentage content of the film-forming material is 20%-60%, wherein the mass percentage content refers to the percentage of the mass of the film-forming material relative to the total mass of the lurasidone hydrochloride oral soluble film composition;
and/or,
a mass percentage content of the plasticizer is 0%-20%, wherein the mass percentage content refers to the percentage of the mass of the plasticizer relative to the total mass of the lurasidone hydrochloride oral soluble film composition;
a mass percentage content of the flavoring agent is 0%-25.0%, wherein the mass percentage content refers to the percentage of the mass of the flavoring agent relative to the total mass of the lurasidone hydrochloride oral soluble film composition.

5. The lurasidone hydrochloride oral soluble film composition according to any one of claims 1 to 4, wherein
the lurasidone hydrochloride oral soluble film composition further comprises a disintegrant or a combination of a disintegrant and one or more of a saliva stimulant, a filler, a colorant, an anti-adherent, and a bacteriostatic agent.

6. The lurasidone hydrochloride oral soluble film composition according to claim 5, wherein:
the disintegrant is one or more of low-substituted hydroxypropylcellulose, crospovidone, croscarmellose sodium, sodium carboxymethyl starch, starch, microcrystalline cellulose, and pregelatinized starch;
and/or,
the saliva stimulant is one or more of tartaric acid, malic acid, and mannitol;
and/or,
the filler is one or more of mannitol, sucrose, glucose, maltose, lactose, sorbitol, xylitol, maltitol, galactitol, erythritol, dextrin, and trehalose;
and/or,
the colorant is one or more of titanium dioxide, pigment, and lake;
and/or,
the anti-adherent is one or more of talc, magnesium stearate, and calcium stearate;
and/or,
the bacteriostatic agent is one or more of methylparaben, ethylparaben, and sodium thiosulfate.

7. The lurasidone hydrochloride oral soluble film composition according to claim 5 or 6, wherein
a mass percentage content of the disintegrant is 0-10.0%, wherein the mass percentage content refers to the percentage of the mass of the disintegrant relative to the total mass of the lurasidone hydrochloride oral soluble film composition;
and/or,
a mass percentage content of the saliva stimulant is 0-10.0%, wherein the mass percentage content refers to the percentage of the mass of the saliva stimulant relative to the total mass of the lurasidone hydrochloride oral soluble film composition; and/or,
a mass percentage content of the filler is 0-10.0%, wherein the mass percentage content refers to the percentage of the mass of the filler relative to the total mass of the lurasidone hydrochloride oral soluble film composition;
and/or,
a mass percentage content of the colorant is 0-5.0%, wherein the mass percentage content refers to the percentage of the mass of the colorant relative to the total mass of the lurasidone hydrochloride oral soluble film composition;
and/or,
a mass percentage content of the anti-adherent is 0-5.0%, wherein the mass percentage content refers to the percentage of the mass of the anti-adherent relative to the total mass of the lurasidone hydrochloride oral soluble film composition;
and/or,
a mass percentage content of the bacteriostatic agent is 0-1.0%, wherein the mass percentage content refers to the percentage of the mass of the bacteriostatic agent relative to the total mass of the lurasidone hydrochloride oral soluble film composition.

8. The lurasidone hydrochloride oral soluble film composition according to any one of claims 1 to 7, wherein
the lurasidone hydrochloride oral soluble film composition is selected from any one of the following formulas:
formula 1: 47.2% lurasidone hydrochloride, 33.0% hydroxypropylcellulose, 9.4% glycerol, 9.4% sucralose, and 1.0% titanium dioxide;
formula 2: 30.0% lurasidone hydrochloride, 45.0% polyvinyl alcohol, 12.0% polyethylene glycol 6000, 5.0% sucralose, 3.5% menthol, 0.9% mannitol, 3.0% lactose, and 0.6% methylparaben;
formula 3: 33.3% lurasidone hydrochloride, 45.8% polyvinyl alcohol, 8.3% glycerol, 3.3% sodium chloride, 4.2% citric acid, 4.3% sucralose, 0.4% essence, and 0.4% menthol;
formula 4: 25.0% lurasidone hydrochloride, 50.0% polyvinyl alcohol, 16.0% glycerol, 2.5% stevioside, 0.5% essence, and 6.0% lactose;
formula 5: 41.3% lurasidone hydrochloride, 24.8% hydroxyethylcellulose, 16.5% pullulan, 8.3% glycerol, 8.3% sucralose, and 0.8% titanium dioxide;
formula 6: 47.2% lurasidone hydrochloride, 28.3% hydroxyethylcellulose, 9.4% pullulan, 4.7% glycerol, 9.4% sucralose, and 1.0% titanium dioxide;
formula 7: 52.1% lurasidone hydrochloride, 20.8% hydroxyethylcellulose, 10.4% pullulan, 5.2% glycerol, 10.4% sucralose, and 1.1% titanium dioxide;
formula 8: 41.7% lurasidone hydrochloride, 25.0% hydroxyethylcellulose, 16.7% pullulan, 5.0% glycerol, 8.3% sucralose, 1.7% menthol, 0.8% talc, and 0.8% titanium dioxide;
formula 9: 38.5% lurasidone hydrochloride, 23.1% hydroxyethylcellulose, 15.4% pullulan, 4.5% glycerol, 7.7% sucralose, 1.5% menthol, 0.8% essence, 7.7% citric acid, and 0.8% talc; and
formula 10: 35.7% lurasidone hydrochloride, 21.4% hydroxyethylcellulose, 14.3% pullulan, 4.3% glycerol, 7.1% sodium carboxymethyl starch, 7.1% sucralose, 1.4% menthol, 0.8% essence, 7.1% citric acid, and 0.8% talc.

9. A method for preparing the lurasidone hydrochloride oral soluble film composition according to any one of claims 1 to 8, comprising the following steps:
1) dissolving the film-forming material in water by heating at 60-70 °C to form a solution;
2) adding other components except for the active drug into the solution obtained in step 1) and stirring to obtain a blank gel solution;
3) placing the active drug in the blank gel solution obtained in step 2), stirring until the active drug is dispersed, and defoaming by stirring under vacuum to obtain a drug-containing gel solution; and
4) coating the defoamed drug-containing gel solution obtained in step 3) on a release film by using a coating machine, heating, drying, and cutting to obtain the lurasidone hydrochloride oral soluble film composition;
wherein preferably, the thickness of the lurasidone hydrochloride oral soluble film composition is 10-300 µm;
and/or,
the lurasidone hydrochloride oral soluble film composition is capable of being completely disintegrated in 1000 mL of simulated saliva at 37±1 °C within 2 min.

10. Use of the lurasidone hydrochloride oral soluble film composition according to any one of claims 1 to 8 for the manufacturing of a medicament for the treatment and/or prevention of depression.
